# EUROPEAN PATENT APPLICATION

(11) **EP 4 302 750 A1**
(43) Date of publication of application: **10.01.2024**
(21) Application number: 22183690.1
(22) Date of filing: 07.07.2022
(51) Int. Cl.: A61K 9/00, A61K 9/70, A61K 31/137, A61P 37/08, A61K 47/32, A61K 47/38, A61K 47/10, A61K 47/34, A61K 47/20

(54) **ORAL THIN FILMS COMPRISING ADRENALINE**

(71) Applicant: LTS Lohmann Therapie-Systeme AG, 56626 Andernach (DE); ALK-Abelló A/S, 2970 Hørsholm (DK)
(72) Inventor: MÜLLER, Markus, 53840 Troisdorf (DE); LINN, Michael, 55596 Waldböckelheim (DE); RICHTER-FRIIS, Martin, 2970 Hørsholm (DK)
(74) Representative: Meissner Bolte Partnerschaft mbB

(57) **Abstract**

Oral thin film comprising one or more layer(s), wherein at least one layer contains adrenaline or a pharmaceutically acceptable salt thereof and at least one polymer, characterized in that the adrenaline is substantially undissolved in the at least one polymer.

Method for preparing said oral thin film, comprising the steps:

a) Preparing a solution comprising said at least one polymer and a solvent in which adrenaline or the pharmaceutically acceptable salt thereof is substantially insoluble,

(b) addition of adrenaline,

(c) spreading and drying the suspension obtained in step (b), in order to obtain a single-layer oral thin film, and

Said oral thin film for use as a medicament, for use as a medicament in the treatment of allergic reactions.

## Description

The present invention is directed to an oral thin film, a process for its preparation and its use as a medicament.

Oral thin films (OTF) are thin films containing at least one pharmaceutically active substance that are placed directly in the oral cavity or applied to the oral mucosa and dissolve there. In particular, these are thin polymer-based films containing active ingredient which, when applied to a mucosa, especially the oral mucosa, release the active ingredient directly into it. The very good blood circulation of the oral mucosa ensures a rapid transfer of the active ingredient into the blood circulation. This dosage system has the advantage that the active ingredient is largely absorbed through the mucosa, thus avoiding the "first pass metabolism" that occurs with the conventional dosage form of an active ingredient in tablet form.

A problem with the administration of adrenaline, especially in dissolved form in an oral thin film, is that the active ingredient gets initially dissolved or partially dissolved in the process of the preparation of the oral thin film and partially recrystallizes when the solvent is evaporated. Recrystallization is undesirable, however, as this causes a morphological change in the active ingredient and can also have a negative effect on the chemical stability of the active ingredient. In particular, an inhomogeneous appearance on the surface can occur, which is shown in spots and/or macroscopically visible crystals/crystal accumulations. In addition, recrystallization can also have a negative effect on the mechanical properties of the oral thin film.

While the degradation of adrenalin to adrenochrome leads to a strong coloration, the degradation mechanisms to D-adrenalin and adrenaline sulfonate are responsible for the quantitatively largest degradation. This happens via a planar sp²-transition state which is stabilized by the activated aromatic. Reactions of the transition state with water lead to the formation of D- and L-adrenaline in a ratio of 1:1, which in the long run leads to a racemization of the starting material. The large use of sodium metabisulfite in the existing market products (Adrenaline^{®} ratio 1:1, Epipene ratio 1.7:1, based on sodium metabisulfite to adrenaline) leads to the reaction of sulfite (SO₃²⁻) with adreanlin (S_{N}1 reaction). At the end of shelf-life, known market products therefore contain up to 15% adrenaline sulfonate.

US 2018/0125977 A1 discloses pharmaceutical compositions for the administration of adrenaline, wherein the adrenaline is partially dissolved in the composition.

The problem underlying the present invention is to overcome the aforementioned disadvantages of the prior art. In particular, the problem of the present invention is to provide an oral thin film for the administration of adrenaline or a pharmaceutically acceptable salt thereof, in which uncontrolled recrystallization of the adrenaline or the pharmaceutically acceptable salt thereof is largely prevented. Furthermore, the adrenaline or the pharmaceutically acceptable salt thereof should also be present in the oral thin film in a chemically largely stable state so that the degradation or racemization of the pharmaceutically active L-adrenaline is prevented without the need to use large amounts of antioxidants, in particular sodium metabisulfite. Furthermore, the oral thin film should be easy and inexpensive to produce.

This problem is solved by an oral thin film according to claim 1 which comprises one or more layer(s), wherein at least one layer contains adrenaline or a pharmaceutically acceptable salt thereof and at least one polymer, characterized in that the adrenaline is substantially undissolved in the at least one polymer. Such a dosage form makes it possible to eliminate the above-mentioned disadvantages. In particular, the adrenaline is crystalline during the entire production process, so that during the production or storage of the oral thin film, uncontrolled or partial recrystallization does not occur, which would result in the above-mentioned disadvantages.

Due to the polymer matrix of the oral thin film, the active ingredient adrenaline is also well protected against oxygen and extrinsic moisture. Since adrenaline in crystalline form is also chemically very stable, this largely prevents the racemization of L-adrenaline, even without having to resort to large amounts of antioxidants, especially sodium metabisulfite.

Substantially undissolved means that more than 95 % by weight, preferably more than 98 % of adrenaline is present undissolved, i.e. crystalline.

Preferably, adrenalin is at least poorly soluble in the at least one polymer at temperatures of 15°C to 25°C. The definition of different grades of solubility is given below.

| Term | V(solvent) in ml substance (g) | g·l⁻¹ solvent |
|---|---|---|
| Very easily soluble | < 1 | >1000 |
| easily suluble | 1 bis 10 | 100 bis 1000 |
| soluble | 10 bis 30 | 33 bis 100 |
| Less soluble | 30 bis 100 | 10 bis 33 |
| poorly soluble | 100 bis 1000 | 1 bis 10 |
| hardly löslich | 1000 bis 10000 | 0.1 bis 1 |
| insoluble | > 10000 | < 0.1 |

The adrenaline is thus preferably present as a solid suspension in the at least one polymer.

Adrenaline or epinephrine is a hormone produced in the adrenal medulla which belongs to the group of catecholamines. Adrenaline is also found in the central nervous system, where it is present as a neurotransmitter in adrenergic nerve cells. Adrenaline mediates its effects by activating G-protein-coupled receptors, the adrenoceptors.

Adrenaline is also known under the systematic IUPAC name 4-[1-hydroxy-2-(methylamino)ethyl]benzene-1,2-diol. Only the L-enantiomer is pharmaceutically active.

The oral thin film according to the invention is therefore preferably characterized in that the adrenaline or the pharmaceutically acceptable salt thereof is present as the L-entatiomer.

The adrenaline is especially preferred as adrenaline hydrogentartrate, especially as L-adrenaline hydrogentartrate.

The oral thin film according to the invention is further preferably characterized in that the adrenaline or the pharmaceutically acceptable salt thereof is present micronized having a particle size distribution of d(99) < 10 µm.

d(99) means that more than 99% of the particles are smaller than 10 µm.

The particle size distribution may be determined by analysis using light microscopy.

The particle size distribution may also be determined according to European Pharmacopoeia (Ph. Eur.) 10th edition.

The particle size distribution is preferably such that the largest particles do not exceed a size of 12 µm and do not fall below a size of 0.5 µm.

The use of micronized active ingredient on the one hand increases the dissolution rate in the final film, which can be advantageous for the absorption, and on the other hand enables processing into a homogeneous film.

The oral thin film according to the invention is further preferably characterized in that the at least one polymer comprises at least one water-soluble polymer.

Water-soluble polymers comprise chemically different, natural or synthetic polymers whose common characteristic is their solubility in water or aqueous media. A prerequisite is that these polymers have a sufficient number of hydrophilic groups for water solubility and are not cross-linked. The hydrophilic groups can be non-ionic, anionic, cationic and/or zwitterionic.

Preferably the at least one polymer in the oral thin film of the invention is selected from the group consisting of starch and starch derivatives, dextrans, cellulose derivatives such as carboxymethyl cellulose, hydroxypropyl cellulose, hydroxyethyl cellulose, hydroxypropylmethyl cellulose, hydroxypropylethyl cellulose, sodium carboxymethyl cellulose, ethyl or propyl cellulose, polyacrylic acids, polyacrylates, polyvinyl pyrrolidones, polyvinyl alcohols, polyvinyl pyrrolidone/polyvinyl acetate copolymers, polyethylene oxide polymers, polyacrylamides, polyethylene glycols, polyvinyl alcohol/polyethylene glycol co-polymers, gelatine, collagen, alginates, pectin, pullulan, tragacanth, chitosan, alginic acid, arabinogalactan, galactomannan, agar, agarose, carrageenan, natural gums and mixtures thereof.

Hydroxypropyl cellulose, polyvinyl pyrrolidones, polyvinyl pyrrolidone/polyvinyl acetate copolymers, polyvinyl alcohol, polyvinyl alcohol/polyethylene glycol co-polymers and/or pectin (amylopectin) are particularly preferred.

The oral thin film according to the invention is preferably characterized in that the oral thin film contains less than 1% by weight, preferably less than 0.5% by weight, more preferably less than 0.1% by weight, based on the total weight of the respective layer of the oral thin film or based on the total weight of the oral thin film, respectively.

Known antioxidants or stabilizers used include in particular tocopherols and their esters, sesamol from sesame oil, coniferous benzoate from benzoin resin, gallates (including methyl, ethyl, propyl, amyl, butyl, lauryl gallate), butylated hydroxyanisole (BHA/BHT, also butyl-p-cresol), ascorbic acid and salts and esters thereof (e.g. acorbyl palmitate), erythorbic acid (isoascorbinic acid) and salts and esters thereof, monothioglycerol, sodium formaldehyde sulfoxylate, sodium metabisulfite, sodium bisulfite, sodium sulfite, potassium metabisulfite, butyl hydroxyanisole, butyl hydroxytoluene (BHT) and/or propionic acid.

The largely or complete absence of antioxidants has the advantage that the antioxidants cannot react with the active ingredient and produce undesirable compounds. Furthermore, antioxidants can trigger allergic reactions. The absence or a reduction of antioxidants is therefore advantageous.

In particular, it is advantageous that the oral thin film of the invention contains less than 1% by weight, preferably less than 0.5% by weight, more preferably less than 0.1% by weight of a metabisulfite, such as sodium metabisulfite or potassium metabisulfite, based on the total weight of the respective layer of the oral thin film or based on the total weight of the oral thin film, respectively.

Especially a low amount or even the absence of sodium metabisulfite is advantageous, as sodium metabisulfite can cause allergic reactions and can react with adrenaline to adrenaline sulfonate.

The oral thin film according to the invention is preferably characterized in that the oral thin film contains less than 5% by weight, preferably less than 4% by weight, preferably less than 3% by weight, preferably less than 2% by weight, preferably less than 1% by weight, preferably less than 0.5% by weight and more preferably less than 0.1% by weight of antimicrobial substances, based on the total weight of the respective layer of the oral thin film or based on the total weight of the oral thin film, respectively.

The absence of antimicrobial substances has the advantage that antimicrobial substances may also be toxic to the patient or may cause allergic reactions. This risk is reduced by dispensing with antimicrobial substances as far as possible.

Known antimicrobial substances comprise especially phyto-extracts.

The oral thin film according to the invention is therefore preferably characterized in that the oral thin film contains less than 5% by weight, preferably less than 4% by weight, preferably less than 3% by weight, preferably less than 2% by weight, preferably less than 1% by weight, preferably less than 0.5% by weight and more preferably less than 0.1% by weight of phyto-extract, based on the total weight of the respective layer of the oral thin film or based on the total weight of the oral thin film, respectively.

The oral thin film according to the invention is preferably characterized in that the oral thin film contains less than 10% by weight, preferably less than 9% by weight, preferably less than 8% by weight, preferably less than 7% by weight, preferably less than 6% by weight, preferably less than 5% by weight and more preferably less than 3% by weight of water, based on the total weight of the respective layer of the oral thin film or based on the total weight of the oral thin film, respectively.

In order to determine the water content, the oral thin film is analyzed via coulometric Karl Fischer titration after dissolution in 5 mL of dimethyl sulfoxide through shaking on a laboratory shaker at about 1200 rpm for 10 min.

The oral thin film according to the invention may however comprise up to 10% by wt., preferably up to 4% by wt. of an organic solvent, such as ethanol and/or acetone, based on the total weight of the respective layer of the oral thin film or based on the total weight of the oral thin film, respectively.

The oral thin film according to the invention is preferably characterized in that the oral thin film contains at least 40% by weight, preferably at least 45% and more preferably at least 50% by weight of adrenaline or a pharmaceutically acceptable salt thereof (as defined above), based on the total weight of the respective layer of the oral thin film or based on the total weight of the oral thin film, respectively.

Preferably, the oral thin film according to the invention is characterized in that the oral thin film contains 40% by weight to 65% by weight, preferably 45% to 60% by weight and more preferably 50% by weight to 56% by weight to of adrenaline or a pharmaceutically acceptable salt thereof (as defined above), based on the total weight of the respective layer of the oral thin film or based on the total weight of the oral thin film, respectively.

The oral thin film of the invention is preferably characterized in that the oral thin film contains at least 30% by weight to 60 % by weight, preferably 33% by weight to 55 % by weight, preferably 35% by weight to 50% by weight, preferably 38% by weight to 44% by weight of the at least one polymer, based on the total weight of the respective layer of the oral thin film or based on the total weight of the oral thin film, respectively.

Blends of several polymers can also be used. In this case the sum of all polymers used is preferably 30% by weight to 60 % by weight, preferably 35% by weight to 55 % by weight, preferably 38% by weight to 51% by weight, preferably 39% by weight to 50 % by weight, based on the total weight of the respective layer of the oral thin film or based on the total weight of the oral thin film, respectively.

The oral thin film according to the invention is preferably characterized in that the oral thin film further comprises at least one excipient selected from the group consisting of dyes, flavors, sweeteners, taste masking agents, emulsifiers, enhancers, pH regulators, humectants and/or preservatives.

The excipients may each be present in the oral thin film in an amount of about 0.001 to 10% by weight, preferably 0.01 to 5% by weight, preferably 0.01 to 2% by weight, based on the total weight of the respective layer of the oral thin film or based on the total weight of the oral thin film, respectively.

The oral thin film according to the invention is further preferably characterized in that the oral thin film has a basis weight of 20 to 400 g/m², preferably of 100 to 350 g/m².

The oral thin film according to the invention is preferably characterized in that the oral thin film comprises at least two layers.

The two layers are preferably identical, but can also have a different composition.

It is also possible that the oral thin film according to the invention has more than two layers. Again, the compositions of the individual layers are either identical or variable.

Preferably the oral thin film of the invention is characterized in that the oral thin film comprises at least two layers, wherein the at least two layers each contain adrenaline or a pharmaceutically acceptable salt thereof and at least one polymer, and in that adrenaline is substantially undissolved in each of the at least one polymers.

The oral thin film according to the invention is further preferably characterized in that the multiple layers are joined by a pharmaceutically acceptable adhesive.

The several layers, preferably two layers, can also be joined by other methods familiar to the person skilled in the art. These include, for example, sealing or heat-sealing, direct coating or laminating of the first layer with a second layer.

The oral thin film according to the invention is preferably characterized in that the multiple layers are joined by a pharmaceutically acceptable adhesive, the pharmaceutically acceptable adhesive preferably comprises at least one water-soluble polymer and at least one plasticizer.

The multilayer oral thin film of the invention is further preferably characterized in that the at least one water-soluble polymer in the adhesive comprises shellac, a vinylpyrrolidone/vinyl acetate copolymer, a polyvinylcaprolactam/polyvinyl acetate/polyethylene glycol copolymer, hydroxypropyl cellulose or hydroxypropylmethyl cellulose and/or polyvinylpyrrolidone.

Preferably, the oral thin film according to the invention is characterized in that the at least one plasticizer in the adhesive comprises glycerol, polyethylene glycol, in particular polyethylene glycol 200, sorbitol and/or tributyl citrate.

In particular, the at least one plasticizer in the adhesive comprises glycerol, polyethylene glycol 200 and/or tributyl citrate.

The use, preferably as a mixture, of at least one water-soluble polymer and at least one plasticizer produces a sticky mixture, which, preferably after drying, can be used as an adhesive layer in the oral thin film according to the invention.

Preferably the weight ratio of the at least one water-soluble polymer to the at least one plasticizer in the adhesive is about 85 to 50 to about 15 to 50, preferably 85 to 65 to about 15 to 35, more preferably about 80 to 60 to about 20 to 40, more preferably about 80 to 50 to about 20 to 50, more preferably about 82 to 68 to about 18 to 32 and most preferably about 80 to 70 to about 20 to 30.

If too little or too much plasticizer is used, the mixture is either not sticky or a processing of the mixture is not possible.

It has been shown that even special mixtures of two polymers are already so tacky that they can be used as adhesives. In this case, a mixture of 5 to 15% by weight of polyvinylpyrrolidone/polyvinyl acetate copolymer and 35 to 45% by weight of hydroxypropyl cellulose has proven to be useful.

The present invention also concerns a process for producing the oral thin film according to the invention. The method comprises the steps:
a) Preparing a solution comprising said at least one polymer and a solvent in which adrenaline or the pharmaceutically acceptable salt thereof is substantially insoluble,
(b) addition of adrenaline or a pharmaceutically acceptable salt thereof,
(c) spreading and drying the suspension obtained in step (b) in order to obtain a single-layer oral thin film.

If a multilayer oral thin film is to be produced, the above process further comprises a step
d) coating the first layer obtained in c) with a pharmaceutically acceptable adhesive and applying a further, preferably a second layer identical to the first, in order to obtain a two-layer oral thin film.

Analogously, additional layers can of course be applied to obtain an oral thin film with any number of layers.

All preferred embodiments described above for the oral thin film apply analogously to the methods according to the invention.

The solvent in which adrenaline or the pharmaceutically acceptable salt thereof is substantially insoluble, preferably comprises ethanol, ethyl acetate, methanol, propanol and/or isopropanol.

These solvents have the advantage that the common polymers are well soluble in them, but adrenaline is essentially not.

Essentially insoluble is understood to mean a solubility of at least poorly soluble in the at least one polymer at temperatures of 15°C to 25°C as defined in the4 table above.

The present invention is also directed to an oral thin film obtainable by the aforementioned method.

All preferred embodiments described above for the oral thin film and the method for obtaining the oral thin film apply analogously to the oral thin film obtainable by the method according to the invention.

The present invention is further directed at an oral thin film, as described above or obtainable by the above-mentioned method, for use as a medicament, in particular in the treatment of allergic reactions, in the treatment of shock conditions, in the use in emergency medicine, e.g. for resuscitation, treatment of respiratory diseases and/or for local vasoconstriction.

The invention is further explained by means of non-restrictive examples.

### Examples

### Example 1:

An oral thin film of the composition listed in Table 1 was prepared.

**Table 1:**

| Material | Function | Amount [wt.-%] |
|---|---|---|
| Adrenaline-hydrogentartrate | Active ingredient | 50.0 |
| Kollidon VA 64¹ | Polymer | 39.9 |
| HPC² | Polymer | 10.0 |
| Sodium metabisulfite | Stabilizer/antioxidant | 0.1 |

| | | |
|---|---|---|
| ¹: Polyvinylpyrrolidone/polyvinyl acetate copolymer (from BASF) ^{2:} Hydroxypropyl cellulose (from Ashland) | | |

HPC was dissolved in ethanol as process solvent. Subsequently, Kollidon VA 64, sodium metabisulfite and adrenaline hydrogentartrate were added.

This suspension was applied to a siliconized liner and dried.

An adhesive of the composition listed in Table 2 was also prepared.

**Table 2:**

| Material | Function | Amount [wt.-%] |
|---|---|---|
| Kollidon VA 64 | Polymer | 80.0 |
| Glycerol | Polymer | 20.0 |

For this purpose Kollidon VA 64was dissolved in ethanol as process solvent. Then glycerol was added.

The coating was applied on a siliconized liner and then dried to obtain an adhesive layer.

A multilayer oral thin film was then produced. For this purpose, a laminate piece of the adhesive layer was applied to a laminate piece of the active ingredient layer and another laminate piece of the active ingredient layer was applied to the other side of the adhesive layer.

Advantages of this multilayer oral thin film include better stability of the adrenaline (see also Example 2), no acetate odour, which often occurs in formulations with dissolved adrenaline, and no uncontrolled recrystallization.

### Example 2:

Single-layer oral thin film 197Adr0100 was prepared analogous to Example 1.

Formulations 197Adr0086, 197Adr0078, 197Adr0091 and 197Adr0084 were prepared as follows:
The production of the oral thin films can be carried out by techniques of mass production known to the person skilled in the art, e.g. by agitating/mixing the contained components by means of agitator motor and suitable stirring tools.

Gas is then added to the resulting mass by stirring, e.g. using a foam whipping machine. The foamy mass may then be coated in a constant layer thickness on a coating carrier by suitable equipment (roller applicator, doctor blade, coating box, etc.).

All temperature-stable web-shaped materials from which the dry film can be removed can serve as coating carrier. This can be achieved by material selection of the coating carrier (different surface tensions between foamed mass and substrate) or by suitable dehesive coatings of the coating carrier with e.g. silicones or fluoropolymers.

The process solvent(s) contained, usually water or mixtures of water and organic, water-miscible solvents, is/are removed by drying. From the solid foam layer thus obtained, the oral thin films can be cut or punched to the appropriate size.

**Table 3:**

| **Material** | **197Adr0086** | **197Adr0078** | **197Adr0091** | **197Adr0084** | **197Adr0100** |
|---|---|---|---|---|---|
| Name | Kollicoat | PVA | Kollicoat + Phentolamin | Kollicoat + Proloc | Suspension |
| Adrenaline hydrogentartrate | 55.00% | 55.00% | 55.00% | 55.00% | 50.00% |
| Kollicoat IR¹ | 43.90% | - | 38.80% | 38.90% | - |
| PVA 4-88² | - | 39.90% | - | - | - |
| Kollidone VA64³ | - | - | - | - | 39.90% |
| HPC⁴ | - | - | - | - | 10.00% |
| Proloc 15⁵ | - | - | - | 5.00% | - |
| Phentolamine | - | - | 5.10% | - | - |
| Glycerol | 1.00% | 5.00% | 1.00% | 1.00% | - |
| Triacetine | - | - | - | - | - |
| Na-metabisulfit | 0.10% | 0.10% | 0.10% | 0.10% | 0.10% |
| Solvent | Aqua purificata | Aqua purificata | Aqua purificata | Aqua purificata | Ethanol |
| Area weight (dry) | 294 g/m² | 294 g/m² | 294 g/m² | 294 g/m² | 323.4 g/m² |
| | | | | | |
| Adrenaline hydrogentartrate mg / OTF size | 72.8 mg / 4.5 cm² | 72.8 mg / 4.5 cm² | 72.8 mg / 4.5 cm² | 72.8 mg / 4.5 cm² | 72.8 mg / 4.5 cm² |

| | | | | | |
|---|---|---|---|---|---|
| ^{1:} Polyvinyl alcohol/polyethylene glycol copolymer (from BASF) ^{2:} Polyvinyl alcohol 4-88 (from Merck) ^{3:} Polyvinylpyrrolidone/polyvinyl acetate copolymer (from BASF) ^{4:} Hydroxypropyl cellulose (from Ashland) ^{5:} Carbomer / Amylopectin (from Henkel) | | | | | |

The formulation 197Adr0100 corresponds to an oral thin film according to the invention.

The other formulations correspond to prototype formulations in which the active ingredient is partially dissolved.

The degradation of adrenalin and the racemization after storage at 40°C were measured. The results are summarized in Tables 4 and 5. The measurement was performed by HPLC and by comparing the relative peak sized of the peak arising from the L- and the D-enantiomer, respectively.

**Table 4:**

| | | 25°C | | | | | | | | | |
|---|---|---|---|---|---|---|---|---|---|---|---|
| | | T = 0 | | T = 6w | | T = 3M | | T = 5M | | T = M | |
| | | Ret.Time | Rel.Area | Ret.Time | Rel.Area | Ret.Time | Rel.Area | Ret.Time | Rel.Area | Ret.Time | Rel.Area |
| | | min | % | min | % | min | % | min | % | min | % |
| 197Adr0100 | L-adr | 23.9 | 99.35 | 16.6 | 99.26 | 17.8 | 99.28 | 17.5 | 99.26 | | |
| | D-adr | 26.7 | 0.65 | 18.3 | 0.68 | 19.5 | 0.71 | 19.3 | 0.68 | | |
| 197Adr0086 | L-adr | 23.8 | 99.28 | 16.6 | 98.63 | 17.7 | 98.58 | 17.5 | 98.75 | | |
| | D-adr | 26.7 | 0.73 | 18.3 | 0.81 | 19.5 | 0.91 | 19.3 | 0.89 | | |
| | | | | 29.6 | 0.43 | 33.8 | 0.35 | 32.8 | 0.25 | | |
| | | | | | | | | | | | |
| 197Adr0078 | | | | 12.6 | 0.22 | 12 | 0.15 | | | | |
| | L-adr | 23.9 | 98.85 | 16.6 | 97.35 | 17.8 | 98.61 | 17.5 | 98.60 | | |
| | D-adr | 26.7 | 1.15 | 18.3 | 1.34 | 19.5 | 1.03 | 19.4 | 1.07 | | |
| | | | | 29.6 | 0.92 | 33.9 | 0.24 | 33.0 | 0.21 | | |
| | | | | | | | | | | | |
| 197Adr0084 | L-adr | 23.9 | 99.24 | 16.6 | 98.75 | 17.8 | 98.66 | 17.6 | 98.69 | | |
| | D-adr | 26.8 | 0.77 | 18.3 | 0.82 | 19.5 | 0.95 | 19.3 | 0.96 | | |
| | | | | | | 33.9 | 0.32 | 33.0 | 0.24 | | |

**Table 5:**

| Batch no. | Formulation | | | 40°C | | | | | | | | | | | |
|---|---|---|---|---|---|---|---|---|---|---|---|---|---|---|---|
| | | | | T = 0 | | T = 2w | | T = 6w | | T = 3M | | T = 5M | | T = 6M | |
| | | | | Ret.Time | Rel.Area | Ret.Time | Rel.Area | Ret.Time | Rel.Area | Ret.Time | Rel.Area | Ret.Time | Rel.Area | Ret.Time | Rel.Area |
| | | | | min | % | min | % | min | % | min | % | min | % | min | % |
| 197Adr0100 | Suspension | | | | | | | | | | | 13.3 | 0.06 | | |
| | | 71 | L-adr | 23.9 | 99.35 | 24.3 | 99.38 | 16.6 | 99.26 | 17.8 | 99.20 | 17.6 | 99.26 | | |
| | | | D-adr | 26.7 | 0.65 | 27.2 | 0.62 | 18.3 | 0.66 | 19.6 | 0.72 | 19.4 | 0.68 | | |
| 197Adr0086 | Kollicoat | 55 | | | | | | 12.6 | 0.11 | | | | | | |
| | | | L-adr | 23.8 | 99.28 | 24.3 | 99.02 | 16.6 | 98.41 | 17.8 | 98.33 | 17.6 | 97.96 | | |
| | | | D-adr | 26.7 | 0.73 | 27.2 | 0.86 | 18.3 | 1.06 | 19.6 | 1.3 | 19.4 | 1.56 | | |
| | | | | | | | | 29.7 | 0.28 | 34.2 | 0.18 | 33.4 | 0.21 | | |
| 197Adr0078 | PVA | 53 | | | | 10.6 | 0.12 | | | | | | | | |
| | | | | | | 18 | 0.15 | 12.9 | 0.13 | 12.1 | 0.15 | 11.8 | 0.20 | | |
| | | | L-adr | 23.9 | 98.85 | 24.2 | 98.30 | 16.6 | 97.73 | 17.9 | 97.62 | 17.7 | 96.96 | | |
| | | | D-adr | 26.7 | 1.15 | 27.2 | 1.40 | 18.3 | 1.58 | 19.6 | 1.89 | 19.4 | 2.33 | | |
| | | | | | | | | 29.7 | 0.31 | 32 | 0.12 | 31.7 | 0.11 | | |
| | | | | | | | | | | 34 | 0.16 | 33.4 | 0.20 | | |
| | | | | | | | | | | | | 11.8 | 0.11 | | |
| 197Adr0084 | Proloc + kollicoat | 54 | L-adr | 23.9 | 99.24 | 24.3 | 99.04 | 16.6 | 98.57 | 17.9 | 98.10 | 17.7 | 97.45 | | |
| | | | D-adr | 26.8 | 0.77 | 27.2 | 0.87 | 18.3 | 1.09 | 19.6 | 1.58 | 19.4 | 2.04 | | |
| | | | | | | | | 29.8 | 0.22 | 34.3 | 0.14 | 33.5 | 0.19 | | |

At 40°C after 5 months (values indicate change with respect to initial value)
197Adr0100: 0.09% reduction, of which 0.03% racemization
197Adr0086: 1.32% reduction, of which 0.83% is racemization
197Adr0078: 1.89% reduction, of which 1.18% racemization
197Adr0084: 1.79% reduction, of which 1.27% is racemization

It is shown that the degradation and racemization is lowest with the OTF according to the invention.

## Claims

1. Oral thin film comprising one or more layer(s), wherein at least one layer contains adrenaline or a pharmaceutically acceptable salt thereof and at least one polymer, **characterized in that** the adrenaline is substantially undissolved in the at least one polymer.

2. Oral thin film according to claim 1, **characterized in that** the adrenaline or the pharmaceutically acceptable salt thereof is present micronized having a particle size distribution of d(99) < 10 µm.

3. Oral thin film according to any of the preceding claims, **characterized in that** the adrenaline or the pharmaceutically acceptable salt thereof is present as L-enantiomer.

4. Oral thin film according to any of the preceding claims, **characterized in that** the at least one polymer is selected from the group consisting of starch and starch derivatives, dextrans, cellulose derivatives such as carboxymethyl cellulose, hydroxypropyl cellulose, hydroxyethyl cellulose, hydroxypropylmethyl cellulose, hydroxypropyl ethyl cellulose, sodium carboxymethyl cellulose, ethyl or propyl cellulose, polyacrylic acids, polyacrylates, polyvinylpyrrolidones, polyvinyl alcohols, polyvinylpyrrolidone/polyvinyl acetate copolymers, polyethylene oxide polymers, polyacrylamides, polyethylene glycols, polyvinyl alcohol/polyethylene glycol co-polymers, gelatine, collagen, alginates, pectin, pullulan, tragacanth, chitosan, alginic acid, arabinogalactan, galactomannan, agar, agarose, carrageenan, natural gums and mixtures thereof.

5. Oral thin film according to any of the preceding claims, **characterized in that** the oral thin film contains less than 1% by weight of antioxidants, in particular less than 1% by weight of a metabisulfite, based on the total weight of the respective layer of the oral thin film or based on the total weight of the oral thin film, respectively.

6. Oral thin film according to any of the preceding claims, **characterized in that** the oral thin film contains at least 40% by weight of adrenaline or a pharmaceutically acceptable salt thereof, based on the total weight of the respective layer of the oral thin film or based on the total weight of the oral thin film, respectively.

7. Oral thin film according to any of the preceding claims, **characterized in that** the oral thin film contains at least 35% by weight of said at least one polymer, based on the total weight of the oral thin film.

8. Oral thin film according to any of the preceding claims, **characterized in that** the oral thin film further comprises at least one excipient selected from the group consisting of dyes, flavors, sweeteners, taste masking agents, emulsifiers, enhancers, pH regulators, humectants and/or preservatives.

9. Oral thin film according to any of the preceding claims, **characterized in that** the oral thin film comprises at least two layers, wherein the at least two layers each contain adrenaline or a pharmaceutically acceptable salt thereof and at least one polymer, **characterized in that** the adrenaline is substantially undissolved in each of the at least one polymers.

10. Oral thin film according to any of the preceding claims, **characterized in that** the multiple layers are joined by a pharmaceutically acceptable adhesive, wherein the pharmaceutically acceptable adhesive preferably comprising at least one water-soluble polymer and at least one plasticizer.

11. Method for preparing an oral thin film according to any one of claims 1 to 11, comprising the steps:
a) Preparing a solution comprising said at least one polymer and a solvent in which adrenaline or the pharmaceutically acceptable salt thereof is substantially insoluble,
(b) addition of adrenaline or a pharmaceutically acceptable salt thereof,
(c) spreading and drying the suspension obtained in step (b), in order to obtain a single-layer oral thin film, and
d) optionally coating the first layer obtained in c) with a pharmaceutically acceptable adhesive and applying a further, preferably a second layer identical to the first, in oder to obtain a two-layer oral thin film.

12. Oral thin film obtainable by the method according to claim 11.

13. Oral thin film according to any one of claims 1 to 10 or 12 for use as a medicament.

14. Oral thin film according to any one of claims 1 to 10 or 12 for use as a medicament in the treatment of allergic reactions.
